# EUROPÄISCHE PATENTSCHRIFT

(11) **EP 2 387 450 B1**
(45) Veröffentlichungstag und Bekanntmachung des Hinweises auf die Patenterteilung: **05.03.2014**
(21) Anmeldenummer: 10700697.5
(22) Anmeldetag: 05.01.2010
(51) Int. Cl.: C01B 21/02, B01D 53/86, B01J 8/02, B01J 23/38, C01B 21/22, A61M 16/00

(54) **VERFAHREN UND VORRICHTUNG ZUR ZERSETZUNG VON LACHGAS**
PROCESS AND DEVICE FOR DECOMPOSING LAUGHING GAS
PROCÉDÉ ET DISPOSITIF POUR LA DÉCOMPOSITION D'OXYDE NITREUX

(30) Priorität: 13.01.2009 DE 102009004431; 18.08.2009 DE 102009037885
(43) Veröffentlichungstag der Anmeldung: 23.11.2011
(73) Patentinhaber: Linde AG, 80331 München (DE)
(72) Erfinder: WENNING, Ulrike, 82049 Pullach (DE); ZANDER, Hans-Jörg, 81479 München (DE); WELLENHOFER, Anton, 82069 Hohenschäftlarn (DE); HOFMANN, Karl-Heinz, 82110 Germering (DE); KORN, Wibke, 81477 München (DE); BERAN, Franz, 81476 München (DE); SCHÖDEL, Nicole, 81477 München (DE); SCHMEHL, Wolfgang, 22525 Hamburg (DE)
(74) Vertreter: Dörries, Hans Ulrich
(86) Internationale Anmeldenummer: PCT/EP2010/000014
(87) Internationale Veröffentlichungsnummer: WO 2010/081643

(56) Entgegenhaltungen:
- EP-A1- 2 165 756
- WO-A1-2006/059506
- WO-A2-02/26355
- JP-A- 55 031 463

## Beschreibung

Die Erfindung betrifft ein Verfahren zur katalytischen Zersetzung von Lachgas in einem lachgashaltigen Gas, wobei das lachgashaltige Gas unter Bildung eines lachgashaltigen Einsatzgases mit einem Verdünnungsgas verdünnt und über einen Wärmeaustauschschritt unter Wärmeaustausch mit einem Abgas und einen Heizschritt zum zeitweisen Heizen des lachgashaltigen Einsatzgases in einen Festbettreaktor zur katalytischen Lachgaszersetzung geführt wird, sowie eine Vorrichtung zur Durchführung des Verfahrens. Ein derartiges Verfahren und derartige Vorrichtungen sind beispielsweise aus der US 72335222, der US 20060008401 oder der WO 2006/059506 bekannt.

Das Distickstoffoxid oder Lachgas ist ein ungiftiges farbloses Gas, das seit langem als Anästhesiemittel oder Analgesiemittel allein oder in Kombination mit anderen Wirkstoffen eingesetzt wird. Darüber hinaus entsteht Lachgas auch bei der Salpetersäureherstellung als Nebenprodukt oder wird in organischen Synthesen eingesetzt. Bei der Verwendung als Anästhesie- bzw. Analgesiemittel besteht ein Problem darin, dass das vom Patienten ausgeatmete Lachgas sich im Behandlungsraum über die bestehenden maximal zulässigen Arbeitsplatzkonzentrationen aufkonzentrieren kann. Das derart aufkonzentrierte Lachgas kann daher am Arbeitsplatz auf längere Zeit hinweg zu einer übermäßigen Belastung des medizinischen Personals führen.

Zusätzlich trägt Lachgas auch im beachtlichen Maß zum Treibhauseffekt und zur Zerstörung der Ozon-Schicht bei. Daher ist aus umwelttechnischen Gesichtspunkten eine Zersetzung des Lachgases vor dem Freisetzen in die Atmosphäre nötig.

Es besteht im Stand der Technik die Nachfrage nach einem Verfahren zur effektiven Zersetzung von Lachgas aus verschiedenen Quellen, sei es aus medizinischen Behandlungen, aus der Salpetersäureproduktion, aus Abgasen verschiedener organischer Synthesen, bei der Reinigung/Entleerung von Gasflaschen oder aus Abgasströmen bei der Lachgasherstellung selbst.

Die oben genannten Patente und Patentanmeldungen befassen sich mit der Aufarbeitung von Lachgas aus medizinischen Anwendungen und schlagen im Wesentlichen vor, das Lachgas, das in mehreren Behandlungs- bzw. Operationsräumen eines Klinikums anfällt, zusammen und in einer gemeinsamen katalytischen Aufbereitung aufzureinigen. Hierfür werden vorzugsweise Katalysatoren eingesetzt, die eine Lachgaszersetzung bei relativ niedrigen Temperaturen, d.h. unter 600 °C, ermöglichen. Verschiedene Adsorber können der katalytischen Aufbereitung vorgeschaltet sein, um Störkomponenten, wie Sevofluran, Desfluran, Isofluran, Halothan oder andere halogenierte Kohlenwasserstoffe abzuscheiden.

Darüber hinaus wurde von den Erfindern in der unveröffentlichten europäischen Patentanmeldung EP 08164753.9 ein Verfahren zur Zersetzung von Lachgas vorgeschlagen, welches vorsieht, das bei einem einzelnen Patienten anfallende Lachgas direkt vor Ort im Behandlungszimmer umzusetzen und so ein aufwändiges Sammeln verschiedener Lachgasströme in dem Klinikum zu vermeiden.

Kommerziell erhältliche Verfahren zur Lachgasentfernung basieren im Stand der Technik überwiegend auf einer thermischen oder katalytischen Zersetzung von Lachgas bei über 800 °C.

Den moderneren katalytischen Verfahren, die bei Temperaturen von unterhalb 600 °C arbeiten, ist das Problem gemeinsam, dass die Zersetzung von Lachgas stark exotherm ist. Das heißt, würde das vom Patienten ausgeatmete Gas ohne weitere Vorbehandlung einer katalytischen Zersetzung unterworfen, so würde sich aufgrund der hohen Lachgaskonzentration von bis zu 70% eine derartig starke Exothermie bzw. Hitzeentwicklung am Katalysator zeigen, so dass dieser irreversibel geschädigt würde. Die gängigen Lachgasentfernungsverfahren, wie sie vorangehend geschildert wurden, haben daher die Gemeinsamkeit, dass der Gasstrom auf eine Lachsgaskonzentration von meist unter 5% mit einem anderen lachgasfreien Gas verdünnt wird. Darüber hinaus sind Maßnahmen vorgesehen, um den Katalysator entsprechend isotherm betreiben zu können. D.h. der Katalysator wird gekühlt bzw. erwärmt.

Der vorliegenden Erfindung liegt die Aufgabe zugrunde, ein Verfahren der eingangs erwähnten Art derart zu verbessern, dass eine flexible Zersetzung von Lachgas in einem lachgashaltigen Gas möglich wird, ohne die Temperatur der Lachgaszersetzung stark zu erhöhen und somit die Katalysatorlebensdauer positiv zu beeinflussen.

Die vorliegende Aufgabe wird verfahrensseitig durch die, im Kennzeichen des Anspruches 1 angegebene, Merkmalskombination gelöst. Weitere vorteilhafte Ausgestaltungen der Erfindung werden in den Unteransprüchen angegeben.

Erfindungsgemäß wird das Verdünnungsgas, welches dem lachgashaltigen Gas zugesetzt wird, getrocknet. Zusätzlich wird dem verdünnten lachgashaltigen Einsatzgas stromaufwärts der katalytischen Lachgaszersetzung zumindest ein Teil des Abgases aus der katalytischen Lachgaszersetzung zugemischt.

Als lachgashaltiges Gas wird im Rahmen der Erfindung jeder lachgashaltige Gasstrom verstanden. Dies kann zum einen von Patienten ausgeatmetes Lachgas im Rahmen einer medizinischen Anwendung oder zum anderen ein Abgasstrom aus der Produktion oder Abfüllung beziehungsweise Flaschenfüllung von Lachgas sein. Die Erfindung ist zur Behandlung von beliebigen Gasströmen geeignet, die Lachgas in einer beliebigen Konzentration enthalten.

Durch das erfindungsgemäße Verfahren wird sichergestellt, dass das lachgashaltige Einsatzgas vor der Zufuhr zur katalytischen Zersetzung des Lachgases keinen oder nur einen geringen Wasseranteil enthält. Die Anspringtemperatur einer katalytischen Lachgaszersetzung liegt bei einem trockenen Gas deutlich unter der Anspringtemperatur eines Gases, welches Wasser enthält. Dies hat zum einen den Vorteil, dass das Einsatzgas der katalytischen Zersetzungsreaktion nicht so stark vorgeheizt werden muss. Zum anderen ist die Temperaturdifferenz zwischen der Anspringtemperatur und der, für den Katalysator bzw. die Apparateteile maximal zulässigen, Temperatur größer. Daher können in der katalytischen Zersetzung auch lachgashaltige Einsatzgase mit einem höheren Anteil an Lachgas verarbeitet werden.

Durch die erfindungsgemäße Verdünnung des lachgashaltigen Gases mit getrocknetem Verdünnungsgas und dem weiteren Zumischen von zumindest eines Teils des Abgases aus der katalytischen Lachgaszersetzung wird sichergestellt, dass das Abgas aus der katalytischen Lachgaszersetzung immer nahezu trocken ist. Durch die Rückführung des nahezu trockenen Abgases aus der katalytischen Lachgaszersetzung und die Zumischung in das lachgashaltige Einsatzgas wird der benötigte Anteil an getrocknetem Verdünnungsgas minimiert. So wird eine sehr stabile Prozessführung ermöglicht. Aus dem zurückgeführten Abgas wird vor der Zumischung zum lachgashaltigen Einsatzgas ein Teil in die Atmosphäre abgegeben.

Durch die erfindungsgemäße Verdünnung des lachgashaltigen Einsatzgases mit getrocknetem Verdünnungsgas und Zumischung von zumindest eines Teils des Abgases aus der katalytischen Lachgaszersetzung kann bei dem erfindungsgemäßen Verfahren eine günstigere und niedrigere Reaktortemperatur bei gleichem Lachgasumsatz als im Stand der Technik verwendet werden. Dadurch können die ausgewählten Werkstoffe an die niedrigere Reaktionstemperatur angepasst werden. Zusätzlich lässt sich das erfindungsgemäße Verfahren bei gleichem Umsatz mit einem geringeren Reaktorvolumen betreiben. Dies ist zusammen mit dem erweiterten Spektrum möglicher Werkstoffe von besonderem Vorteil für die Anwendung zur Lachgaszersetzung in einer Klinik. Durch das erfindungsgemäße Verfahren kann die Lachgaszersetzung in kleinen und kompakten Apparaten direkt in einem Behandlungsraum durchgeführt werden.

Nach dem erfindungsgemäßen Verfahren wird durch das Verdünnungsgas der Feuchtegehalt im gesamten Gasstrom, der als Einsatzstrom in die katalytische Lachgaszersetzung geführt wird, konstant gehalten. Durch die Verdünnung des lachgashaltigen Gases mit dem getrockneten Verdünnungsgas und durch die Zumischung von zumindest eines Teils des Abgases darf das lachgashaltige Gas selbst auch Feuchte bis zur Sättigung enthalten. Der Einsatzstrom für die katalytische Lachgaszersetzung ist immer nahezu trocken. Dies führt zu einer stabilen Reaktionsführung und somit zu minimalen Mess-, Steuer- und Regelungsaufwand. Dadurch werden der Gesamtwirkungsgrad und damit die Wirtschaftlichkeit des Verfahrens erhöht.

Die genannten Vorteile des erfindungsgemäßen Verfahrens stellen sich sowohl bei isothermer, als auch bei adiabater Reaktionsführung der katalytischen Lachgaszersetzung ein.

Durch das erfindungsgemäße Verfahren wird die Flexibilität der katalytischen Lachgaszersetzung deutlich erhöht. Zum einen ist die Variationsbreite bezüglich einer Temperaturerhöhung durch einen höheren Anteil an Lachgas im lachgashaltigen Einsatzgas durch die nach dem erfindungsgemäßen Verfahren herabgesetzte Reaktionstemperatur deutlich größer. Zum anderen lässt sich die Konzentration des Lachgases in dem lachgashaltigen Einsatzgas vor der katalytischen Lachgaszersetzung leicht über die Zufuhr des getrockneten Verdünnungsgases regulieren. Durch die erfindungsgemäße Kombination der Zufuhr von getrocknetem Verdünnungsgas und der Zumischung von zumindest eines Teils des Abgases aus der katalytischen Lachgaszersetzung wird im Normalbetrieb des Verfahrens nur eine geringe Menge getrockneten Verdünnungsgases benötigt. Diese Menge kann bei erhöhter Konzentration des Lachgases im lachgashaltigen Einsatzgas einfach erhöht werden.

Vorteilhafterweise werden als Katalysator Edelmetallkatalysatoren auf Trägermaterialen für die Umsetzung nach dem erfindungsgemäßen Verfahren verwendet. Zu den vorteilhaften Edelmetallen zählen hierbei Palladium, Rhodium, Platin und Rhutenium, wobei Palladium und Rhodium besonders bevorzugt sind. Als Trägermaterialien werden zweckmäßigerweise im Stand der Technik bekannte Materialien wie z.B. Aluminiumoxid, Siliziumoxid oder Zeolite verwendet.

Bevorzugt wird Luft oder ein Inertgas als Verdünnungsgas verwendet. Luft ist kostengünstig und unbegrenzt verfügbar. Ebenso wie die bevorzugten Inertgase beeinflusst Luft die katalytische Lachgaszersetzungsreaktion nicht. Daher sind Luft oder Inertgas als Verdünnungsgas hervorragend geeignet.

Bevorzugt wird das Verdünnungsgas so weit getrocknet, dass das lachgashaltige Einsatzgas einen Taupunkt von -20°C, besonders bevorzugt -40°C, aufweist. Ein Feuchtegehalt im lachgashaltigen Einsatzgas, der einem Taupunkt von -20°C, bevorzugt -40°C, entspricht, ist ausreichend, um die vollen Vorteile des erfindungsgemäßen Verfahrens zu nutzen.

In einer Ausgestaltung der Erfindung erfolgt die Zumischung des Abgases in das lachgashaltige Einsatzgas stromaufwärts des Wärmeaustauschschrittes und des Wärmeaustausches mit dem Abgas. In dieser Ausgestaltung der Erfindung wird das Abgas aus der katalytischen Lachgaszersetzung über einen Wärmeaustauscher geführt, bevor es dem lachgashaltigen Einsatzstrom zugemischt wird. Der verdünnte lachgashaltige Einsatzstrom mit dem zugemischten Abgas wird dann über denselben Wärmeaustauscher in den Reaktor zur katalytischen Lachgaszersetzung geführt. Somit wird in dieser Ausgestaltung der Erfindung ein Wärmeaustauscher zur Abkühlung des Abgases und gleichzeitigen Anwärmung des lachgashaltigen Einsatzgases verwendet.

Alternativ kann das heiße Abgas zumindest teilweise auch direkt in das lachgashaltige Einsatzgas geführt werden.

In einer anderen Ausführung der Erfindung wird das lachgashaltige Einsatzgas nach der Zuführung der Verdünnungsluft zum lachgashaltigen Gas getrocknet.

Die Trocknung des Verdünnungsgases erfolgt zweckmäßigerweise mit einem Einmal-Trockner, einem Molsieb, einem Adsorber, einem Wechselbett-Adsorber oder einem Membrantrockner. Die genannten Mittel zur Trocknung des Verdünnungsgases sind im Stand der Technik etabliert und weisen unterschiedliche Vorteile auf. Einmaltrockner sind etablierte und günstige Mittel zur Trocknung eines Einsatzgases. Durch das erfindungsgemäße Verfahren muss nur eine geringe Menge an Verdünnungsgas getrocknet werden. Daher ist es auch ausreichend, den Einmaltrockner in längeren Zeitabständen auszutauschen. Adsorber oder Molsiebe müssen regeneriert werden. Durch die geringe Menge des zu trocknenden Verdünnungsgases ist auch diese Regeneration nur in längeren Zeitabständen erforderlich. Um einen kontinuierlichen Betrieb des Verfahrens zu gewährleisten, können Wechselbett-Adsorber verwendet werden. Dabei wird ein Adsorber zur Trocknung des Verdünnungsgases verwendet, während gleichzeitig ein weiterer Adsorber regeneriert wird. In einer vorteilhaften Ausgestaltung wird heißes Abgas aus der katalytischen Lachgaszersetzung zur Regeneration der/des Adsorber/s verwendet.

In einer anderen Ausgestaltung der Erfindung kann zusätzlich noch das lachgashaltige Gas vor der Verdünnung einem separaten Schritt zur Trocknung oder zur Reinigung von halogenhaltigen Verbindungen zugeführt werden.

Vorteilhafterweise wird die Lachgaskonzentration im lachgashaltigen Einsatzgas vor und/oder nach der Verdünnung mit dem Verdünnungsgas und/oder direkt vor dem Reaktor gemessen. Auf Basis dieser Messwerte können Reaktionstemperatur, Temperatur des lachgashaltigen Einsatzgases, zugemischte Menge Verdünnungsgas und/oder zugemischte Menge Abgas reguliert werden. Zur Steuerung der Temperatur des lachgashaltigen Einsatzgases lässt sich auch vorteilhafterweise ein Bypass um den Wärmetauscher zum Wärmeaustausch zwischen lachgashaltigen Einsatzgas und heißem Abgas verwenden.

In einer Ausgestaltung der Erfindung werden in mehrere lachgashaltige Einsatzströme in einem Gasbehälter gesammelt und von dort als Einsatzstrom in das erfindungsgemäße Verfahren geführt. Diese Ausgestaltung der Erfindung ist besonders für Produktionsanlagen, Lachgastanks oder Flaschenabfüllungen geeignet. In derartigen Anwendungsfällen existieren zumeist Abgasleitungen für Lachgashaltige Gasströme. In dieser Ausgestaltung der Erfindung werden die existierenden Leitungen mit einem Niederdruck Gasbehälter verbunden. In dem Niederdruckgasbehälter werden alle lachgashaltigen Gasströme gesammelt. Aus dem Gasbehälter wird dann ein lachgashaltiges Gas entnommen und als Einsatzstrom in das erfindungsgemäße Verfahren geführt. Auf diese Weise können auch Schwankungen in der Menge der einzelnen Gasströme durch den Gasbehälter abgefangen werden.

Vorrichtungsseitig wird die gestellte Aufgabe durch die kennzeichnenden Merkmale des Anspruches 8 gelöst.

Die erfindungsgemäße Vorrichtung zur katalytischen Zersetzung von Lachgas umfasst eine Zuführung des Lachgases zu einem Verdichter, einen Wärmetauscher, einen Heizer und einen Festbettreaktor, welcher einen Katalysator aufweist und zur katalytischen Zersetzung von Lachgas geeignet ist, sowie eine Zuleitung in die Zuführung zum Verdichter. Erfindungsgemäß weist die Vorrichtung ferner eine strömungstechnische Verbindung zwischen dem Abgasauslaß des Festbettreaktors und der Zuführung zum Verdichter auf, welche über den Wärmetauscher führt. Erfindungsgemäß ist die Zuleitung mit einem Trockner verbunden, welcher zur Trocknung eines Verdünnungsgases geeignet ist..

Mit der vorliegenden Erfindung gelingt es insbesondere eine stabile Verfahrensführung zu gewährleisten. Auf unterschiedliche Konzentrationen von Lachgas in dem lachgashaltigen Einsatzgas kann dabei flexibel reagiert werden. Die Anspringtemperatur und die Reaktionstemperatur der katalytischen Lachgaszersetzung kann gegenüber einem Verfahren nach dem Stand der Technik deutlich geringer gewählt werden.

Im Folgenden soll die Erfindung anhand eines in der Figur dargestellten Ausführungsbeispieles näher erläutert werden.

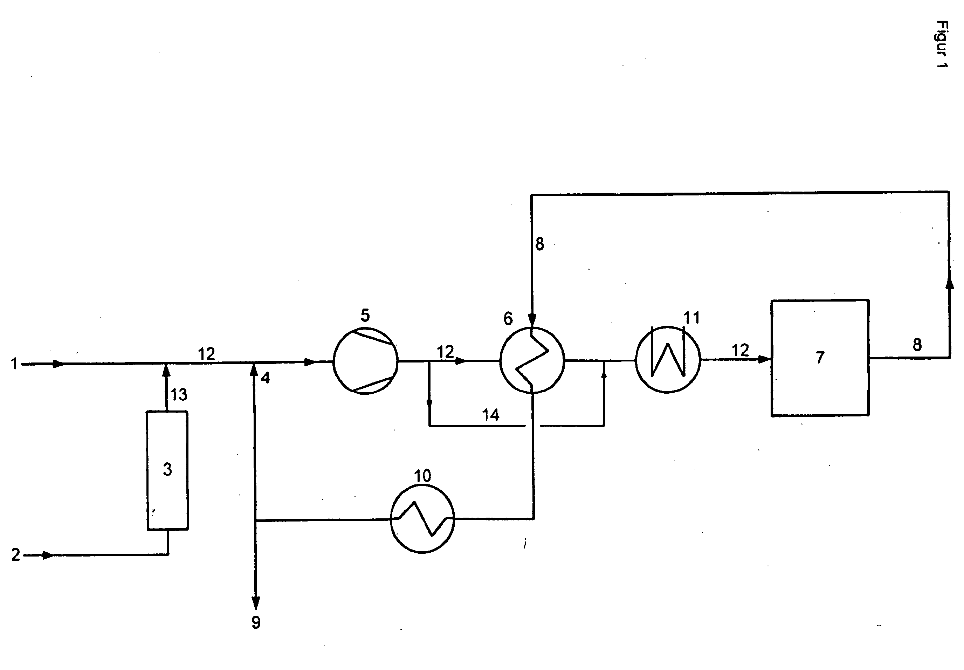

Es zeigt
- Figur 1: ein Ausführurigsbeispiel des erfindungsgemäßen Verfahrens und der erfindungsgemäßen Vorrichtung

Figur 1 zeigt ein Ausführungsbeispiel des erfindungsgemäßen Verfahrens und der erfindungsgemäßen Vorrichtung zur katalytischen Zersetzung 7 von Lachgas in einem lachgashaltige Gas 1. Das lachgashaltige Gas 1 wird mittels eines Verdünnungsgases 2 verdünnt. Erfindungsgemäß wird das Verdünnungsgas 2 in dem Trockner 3 von Wasseranteilen nahezu befreit. In dieser Ausgestaltung der Erfindung wird Luft als Verdünnungsgas 2 verwendet. Nach der Zuführung des Verdünnungsgases 2 über die Zuleitung 13 wird dem lachgashaltigen Einsatzgas 12 Abgas 8 aus der katalytischen Zersetzung 7 zugemischt 4. Nach der Zumischung 4 des Abgases 8 aus der katalytischen Lachgaszersetzung 7 wird das lachgashaltige Einsatzgas 12 verdichtet 5, und in den Wärmeaustauscher 6 geführt. Im Wärmeaustauscher 6 wird das lachgashaltige Einsatzgas 12 durch Wärmeaustausch mit dem Abgas 8 vorgewärmt. Das Abgas 8 wird dabei im Wärmeaustauscher 6 abgekühlt. Das vorgewärmte lachgashaltige Einsatzgas 12 wird über einen weiteren Heizer 11 als Einsatz in die katalytischen Lachgaszersetzung 7 geführt. Der Heizer 11 wird nur zur zusätzlichen Vorheizung des lachgashaltigen Einsatzstromes 12 verwendet. Die Eintrittstemperatur in der katalytischen Lachgaszersetzung liegt in dieser Ausgestaltung der Erfindung ungefähr bei 350 Grad °C. Die katalytische Lachgaszersetzung findet dabei bei Atmosphärendruck oder wenig erhöhtem Druck statt. Ein Teil des Abgases 8 wird aus dem Prozess ausgeführt 9. Das Abgas 8 wird durch einen weiteren optionalen Wärmetauscher 10 weiter abgekühlt. Durch die zusätzliche Abkühlung mittels des Wärmetauschers 10 wird sichergestellt, dass nachfolgende Geräte wie der Verdichter vor Überhitzung geschützt werden. Das dargestellte Ausführungsbeispiel ist für den Einsatz in einem Behandlungsraum und für die Zersetzung von Lachgas aus einer medizinischen Behandlung geeignet. Durch den Wärmetauscher 10 kann hier auch die zulässige Temperatur des Teils des Abgases 8 kontrolliert werden, der an die Atmosphäre abgegeben wird 9. Zur optimalen Steuerung der Eintrittstemperatur des lachgashaltigen Einsatzgases 12 in die katalytische Zersetzung 7 kann zumindest ein Teil des lachgashaltigen Einsatzgases 12 am Wärmetauscher 6 mit einem Bypass 14 vorbeigeführt werden.

## Patentansprüche

1. Verfahren zur katalytischen Zersetzung (7) von Lachgas in einem lachgashaltigen Gas (1), wobei das lachgashaltige Gas (1) unter Bildung eines lachgashaltigen Einsatzgases (12) mit einem Verdünnungsgas (2) verdünnt und über einen Wärmeaustauschschritt (6) unter Wärmeaustausch mit einem Abgas (8) und einen Heizschritt (11) zum zeitweisen Heizen des lachgashaltigen Einsatzgases (12) in einen Festbettreaktor zur katalytischen Lachgaszersetzung (7) geführt wird, **dadurch gekennzeichnet, dass** das Verdünnungsgas (2), welches dem lachgashaltigen Gas (1) zugesetzt wird, getrocknet (3) und dem lachgashaltigen Einsatzgas (12) stromaufwärts der katalytischen Lachgaszersetzung (7) zumindest ein Teil des Abgases (8) aus der katalytischen Lachgaszersetzung zugemischt (4) wird.

2. Verfahren nach Anspruch 1, **dadurch gekennzeichnet, dass** luft oder ein Intergas als Verdünnungsgas (2) verwendet wird.

3. Verfahren nach Anspruch 1 oder 2, **dadurch gekennzeichnet,dass** das Verdünnungsgas so weit getrocknet wird, dass das lachgashaltige Einsatzgas einen Taupunkt von -20°C, Bevorzugt -400°C, aufweist.

4. Verfahren nach einem der Ansprüche 1 bis 3,**dadurch gekennzeichnet, dass** die Zumischung (4) des Abgases (8) in das lachgashaltige Einsatzgas (12) stromaufwärts des Wärmeaustauschschrites (6) und des Wärmeaustausches mit dem Abgas (8) erfolgt.

5. Verfahren nach einem der Ansprüche1 bis 4, **dadurch gekennzeichnet, dass** Trocknung (3) des Verdünnungsgases (2) mit einem Einmal-Trockner, einem Molsieb, einem Adsorber, einem Wechselbett-Adsorber oder einem Membrantrockner erfolgt.

6. Verfahren nach einem der Ansprüche 1 bis 5, **dadurch gekennzeichnet, dass** zumindest ein teil des Abgases (8) direkt in das lachgashaltige Einsatzgas (12) geführt wird.

7. Verfahren nach einem der Ansprüche 1 bis 6, **dadurch gekennzeichnet, dass** mehrere lachgashaltige Gasströme in einem Gasbehälter gesammelt und diesem als ein lachgashaltiges Gas (1) entnommen werden.

8. Vorrichtung zur katalytischen Zersetzung von Lachgas (1) umfassend eine Zuführung (12) des lachgashaltigen Einsatzgases zu einem Verdichter (5), einen Wärmetauscher (6), einen Heizer (11) und einen Festbettreaktor (7), welcher einen Katalysator aufweist und zur katalytischen Zersetzung von Lachgas (1) geeignet ist, sowie eine Zuleitung (13) in die Zuführung zum Verdichter, **dadurch gekennzeichnet, dass** die Vorrichtung eine strömungstechnische Verbindung (8) zwischen dem Abgasauslaß des Festbettreaktors (7) und der Zuführung (12) zum Verdichter (5) aufweist, welche über den Wärmetauscher (6) führt, und die Zuleitung (13) mit einem Trockner (3), geeignet zur Trocknung eines Verdünnungsgases (2), verbunden ist.

## Claims

1. A method for catalytic decomposition (7) of nitrous oxide in a gas (1) containing nitrous oxide, wherein the gas (1) containing nitrous oxide is diluted with a diluting gas (2), thereby forming a nitrous-oxide-containing feedstream gas (12), which is sent to a fixed bed reactor for catalytic decomposition (7) of the nitrous oxide by way of a heat exchanger step (6) involving heat exchange with an exhaust gas (8) and a heating step (11) for temporarily heating the nitrous-oxide-containing feedstream gas (12), **characterized in that** the diluting gas (2), which is added to the nitrous-oxide-containing gas (1), is dried (3), and at least a portion of the exhaust gas (8) from catalytic decomposition of the nitrous oxide is added (4) to the feedstream gas (12) containing nitrous oxide upstream of the catalytic decomposition (7) of nitrous oxide.

2. The method according to Claim 1, **characterized in that** air or an inert gas is used as the diluting gas (2).

3. The method according to Claim 1 or 2, **characterized in that** the diluting gas is dried to such an extent that the nitrous-oxide-containing feedstream gas has a dew point of -20°C, preferably -40°C.

4. The method according to any one of Claims 1 through 3, **characterized in that** the exhaust gas (8) is added (4) to the nitrous-oxide-containing feedstream gas (12) upstream of the heat exchange step (6) and the heat exchange with the exhaust gas (8).

5. The method according to any one of Claims 1 through 4, **characterized in that** the diluting gas (2) is dried (3) with a single-use dryer, a molecular sieve, an adsorber, a moving bed adsorber or a diaphragm-type dryer.

6. The method according to any one of Claims 1 through 5, **characterized in that** at least one portion of the exhaust gas (8) is sent directly into the nitrous-oxide-containing feedstream gas (12).

7. The method according to any one of Claims 1 through 6, **characterized in that** multiple nitrous-oxide-containing gas streams are collected in a gas tank and are withdrawn from this tank as a nitrous-oxide-containing gas (1).

8. An apparatus for catalytic decomposition of nitrous oxide (1) comprising a supply (12) of the nitrous-oxide-containing feedstream gas to a compressor (5), a heat exchanger (6), a heater (11) and a fixed-bed reactor (7), which has a catalyst and is suitable for catalytic decomposition of nitrous oxide (1) as well as an inlet line (13) into the supply to the compressor, **characterized in that** the apparatus has a fluidic connection (8) between the exhaust gas outlet of the fixed-bed reactor (7) and the supply (12) to the compressor (5) leading beyond the heat exchanger (6), and the inlet line (13) is connected to a dryer (3) suitable for drying a diluting gas (2).

## Revendications

1. Procédé de décomposition (7) catalytique de gaz hilarant dans un gaz (1) contenant du gaz hilarant, lors duquel on dilue le gaz (1) contenant du gaz hilarant avec un gaz de dilution (2) en formant une charge gazeuse (12) contenant du gaz hilarant et par l'intermédiaire d'une étape d'échange thermique (6), par échange thermique avec un gaz perdu (8) et d'une étape de chauffage (11) pour le chauffage temporaire de la charge gazeuse (12) contenant du gaz hilarant, on le conduit dans un réacteur à lit fixe pour la décomposition (7) catalytique du gaz hilarant, **caractérisé en ce qu'**on fait sécher (3) le gaz de dilution (2) que l'on ajoute au gaz (1) contenant du gaz hilarant et en amont de la décomposition (7) catalytique de gaz, on ajoute en mélangeant (4) à la charge gazeuse (12) contenant du gaz hilarant au moins une partie du gaz perdu (8) issu de la décomposition catalytique de gaz hilarant.

2. Procédé selon la revendication 1, **caractérisé en ce qu'**on utilise de l'air ou un gaz inerte en tant que gaz de dilution (2).

3. Procédé selon la revendication 1 ou 2, **caractérisé en ce qu'**on fait sécher le gaz de dilution jusqu'à ce que la charge gazeuse contenant du gaz hilarant présente un point de condensation de -20°C, de préférence de -40°C.

4. Procédé selon l'une quelconque des revendications 1 à 3, **caractérisé en ce que** l'ajout par mélange (4) du gaz perdu (8) dans la charge gazeuse (12) contenant du gaz hilarant s'effectue en amont de l'étape d'échange thermique (6) et de l'échange thermique avec le gaz perdu (8).

5. Procédé selon l'une quelconque des revendications 1 à 4, **caractérisé en ce que** le séchage (3) du gaz de dilution (2) s'effectue à l'aide d'un séchoir à passage unique, d'un tamis moléculaire, d'un adsorbant, d'un adsorbant à lit alterné ou d'un séchoir à membrane.

6. Procédé selon l'une quelconque des revendications 1 à 5, **caractérisé en ce qu'**on conduit au moins une partie du gaz perdu (8) directement dans la charge gazeuse (12) contenant du gaz hilarant.

7. Procédé selon l'une quelconque des revendications 1 à 6, **caractérisé en ce qu'**on collecte plusieurs flux gazeux contenant du gaz hilarant dans un collecteur de gaz et on le prélève dans ce dernier en tant que gaz (1) contenant du gaz hilarant.

8. Dispositif pour la décomposition catalytique de gaz hilarant (1) comprenant une arrivée (12) de la charge gazeuse contenant du gaz hilarant vers un condensateur (5), un échangeur thermique (6), un dispositif de chauffage (11) et un réacteur à lit fixe (7), lequel comporte un catalyseur et est adapté pour la décomposition catalytique de gaz hilarant (1), ainsi qu'un conduit d'alimentation (13) dans l'arrivée vers le condensateur, **caractérisé en ce que** le dispositif comporte une liaison (8) technique par écoulement entre la sortie du gaz perdu du réacteur à lit fixe (7) et l'arrivée (12) vers le condensateur (5) qui mène par l'intermédiaire de l'échangeur thermique (6), et **en ce que** le conduit d'alimentation (13) est relié avec un séchoir (3), adapté pour le séchage d'un gaz de dilution (2).
